(19) Europäisches Patentamt
European Patent Office
Office européen des brevets

(11) Veröffentlichungsnummer: **0 353 416**
**A2**

## (12) EUROPÄISCHE PATENTANMELDUNG

(21) Anmeldenummer: **89109828.7**

(22) Anmeldetag: **31.05.89**

(51) Int. Cl.⁴: **A61K 31/10**

Die Bezeichnung der Erfindung wurde geändert (Richtlinien für die Prüfung im EPA, A-III, 7.3).

(30) Priorität: **29.06.88 DE 3821964**

(43) Veröffentlichungstag der Anmeldung:
**07.02.90 Patentblatt 90/06**

(84) Benannte Vertragsstaaten:
**BE CH DE FR GB IT LI NL SE**

(71) Anmelder: **Gesellschaft für Biotechnologische Forschung mbH (GBF) Mascheroder Weg 1 D-3300 Braunschweig-Stöckheim(DE)**

(72) Erfinder: **Baydoun, Hassan, Dr.**
**Jakobstrasse 1a**
**D-3300 Braunschweig(DE)**
Erfinder: **Wagner, Karl G., Prof. Dr.**
**Hoffmann v. Fallerslebenstrasse 18a**
**D-3340 Wolfenbüttel(DE)**
Erfinder: **Wagner, Roland, Dr.**
**Mascheroder Weg 1**
**D-3300 Braunschweig(DE)**
Erfinder: **Plank-Schumacher, Karin**
**Kirchgasse 3**
**D-3344 Ohrum(DE)**
Erfinder: **Scharfenberg, Klaus**
**Lortzingstrasse 19**
**D-3300 Braunschweig(DE)**
Erfinder: **Jain, M.K., Dr.**
**University of Delaware**
**Newark Delaware 19716(US)**
Erfinder: **Apitz-Castro, Rafael, Dr. Laboratorio de Thrombosis**
**Experimental Centro de Biofisica y Bioquimica IVIC**
**Caracas 1010-A(VE)**

(74) Vertreter: **Boeters, Hans Dietrich, Dr. et al**
**Boeters & Bauer, Patentanwälte**
**Thomas-Wimmer-Ring 14**
**D-8000 München 22(DE)**

(54) Verwendung von Ajoen und Ajoen-Derivaten als Cytostatika.

(57) Die Erfindung betrifft die Verwendung von Ajoen und Ajoen-Derivaten als oder in Cytostatika und/oder Cytoxika sowie ein Mittel mit einem Gehalt an den genannten Verbindungen.

## Verwendung von Ajoen und Ajoen-Derivaten als oder in Cytostatika und/oder Cytotoxika sowie ein Mittel mit den Verbindungen.

Es ist bereits bekannt, daß sich bei Tumorzellen eine Immunität erreichen läßt, wenn man sie mit Knoblauchextrakt behandelt; vgl. Nature, 216 (1967) 83 bis 84 und IUPAC Papers, Kyoto 1988, PC 51 und PC 52. In der zuerst genannten Arbeit wird nicht ausgeschlossen, daß dieser Effekt dem Allicin (Allylthiosulfinallylester) zuzuschreiben ist, das im Knoblauchextrakt vorliegt; loc. cit. In Cancer Research, 18 (1958) 1301 bis 1308 wird dieser Effekt direkt für Allicin und die homologen Alkylverbindungen der Formel Alk-SO-S-Alk (Alk = Methyl, Ethyl, Propyl und Butyl) nachgewiesen.

Ferner ist die antithrombotische Wirkung von Ajoen intensiv untersucht worden; vgl. beispielsweise J. Am. Chem. Soc., 106 (1984) 8295 bis 8296. Der genannten Arbeit (Schema I Zeile 3) sowie TIBS, 12 (1987) 252 bis 254 läßt sich auch entnehmen, daß sich Allicin in Ajoen überführen läßt. Die antithrombotische Wirkung ist ferner in folgenden Arbeiten angesprochen: Thrombosis Research, 32 (1983) 155 bis 169, Scientific American, 252 (1985) 94 bis 99, J. Am. Chem. Soc., 108 (1986) 7045 bis 7055 und Biochem. Biophys. Res. Comm. 141 (1986) 145 bis 150.

Obgleich seit der Feststellung, daß Allicin eine cytostatische bzw. cytotoxische Wirkung besitzt, nun 30 Jahre vergangen sind, scheint von dieser Feststellung keine Anregung ausgegangen zu sein, weiter über Allicin zu arbeiten oder im Knoblauchextrakt oder unter Folgeprodukten des Allicins nach weiteren Verbindungen mit entsprechender Wirkung zu suchen. Das kann darauf zurückzuführen sein, daß der Knoblauchextrakt eine ganze Reihe von Inhaltsstoffen enthält (vgl. beispielsweise TIBS, 12 (1987) 252 bis 254) oder daß das Ausmaß des genannten Effekts nicht zu weiteren Untersuchungen ermutigte.

Das allgemeine und große Bedürfnis nach Cytostatika und Cytotoxika ist bekannt. Überraschenderweise wurde nun festgestellt, daß Verbindungen der allgemeinen Formel

$R^1\text{-}CH_2\text{-}SX\text{-}CH_2\text{-}CR^2 = CR^3\text{-}SY\text{-}SZ\text{-}CH_2\text{-}R^4$

worin

$R^1$ und $R^4$ jeweils eine $C_{1-4}$-Alkyl-, Vinyl- oder Phenylgruppe sind,

$R^2$ und $R^3$ Wasserstoffatome sind oder mit den Kohlenstoffatomen, an die sie gebunden sind, einen Phenylring bilden,

x ein oder zwei Sauerstoffatome bedeutet und

Y und Z unabhängig voneinander fehlen können oder ein Sauerstoffatom bedeuten,

eine cytostatische und cytotoxische Wirkung besitzen und sich demgemäß als Cytostatikum und/oder Cytotoxikum oder in Cytostatika und/oder Cytotoxika verwenden lassen.

Eine Ausführungsform der Erfindung ist auf diese Verwendung gerichtet.

Eine weitere Ausführungsform der Erfindung betrifft ein thera peutisches Mittel mit einem Gehalt an den genannten Verbindungen sowie einem pharmazeutisch verträglichen Alkohol, beispielsweise Glycerin, Ether und/oder Etheralkohol. Bei dem Alkohol, Ether bzw. Etheralkohol kann es sich auch um ein Oligomeres oder Polymeres handeln. Das Mittel kann übliche Hilfsmittel enthalten, insbesondere übliche Träger und/oder Verdünnungsmittel, beispielsweise Wasser.

Nachstehend wird die Erfindung durch Beispiele anhand einer Figur näher erläutert.

## Beispiel 1

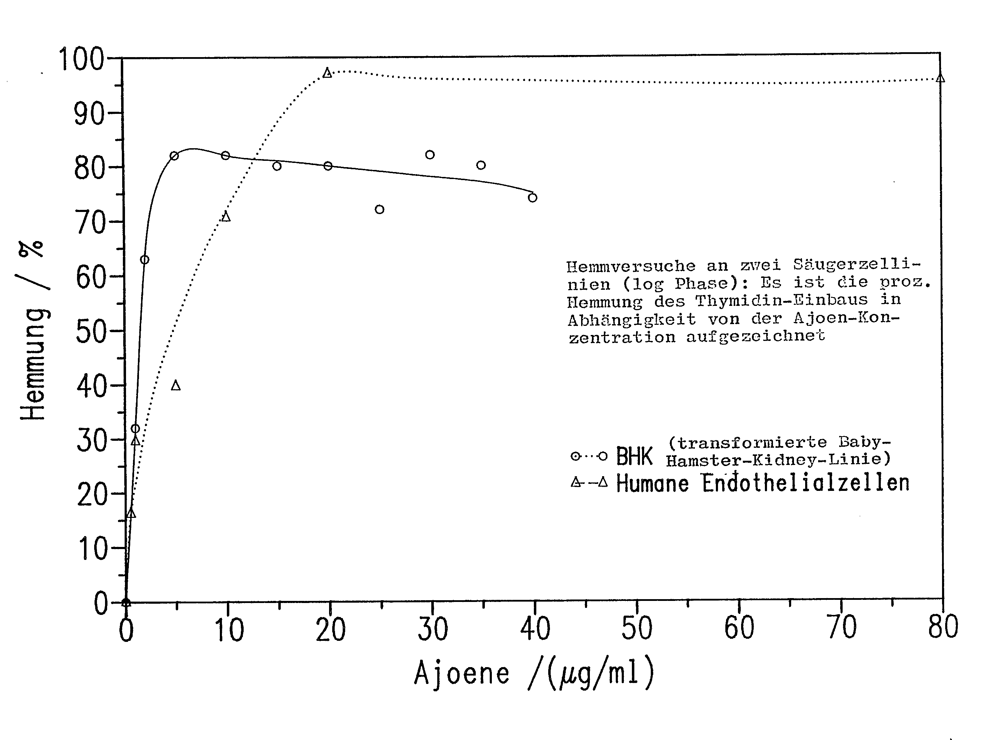

Man ließ Ajoen auf eine Zellkultur der SV40-transformierten Baby-Hamster-Kidney-Linie (BHK; Fibroblast) einwirken. Die Proliferationshemmung wurde über den Einbau von radioaktivem Thymidin getestet. Sie ließ sich ab 1 μg Ajoen/ml ermitteln. Die Figur zeigt eine typische Hemmkurve. Der Figur läßt sich entnehmen, daß die Ki-Werte (50 %) im Bereich von etwa 1 bis 10 und insbesondere etwa 2 bis 5 μg Ajoen/ml lagen.

Die Hemmung der Proliferation ist bis zu etwa 20 μg Ajoen/ml reversibel. Jenseits dieses Wertes wurde eine irreversible, d. h. toxische Wirkung ermittelt.

## Beispiel 2

Es wurde Beispiel 1 mit der Abweichung wiederholt, daß man die Linie einer primären Endothelialzelle verwendete. Die Proliferationshemmung ist in der Figur grafisch dargestellt.

## Beispiele 3 bis 5

Beispiel 1 wurde ferner abweichend mit folgenden Linien nachgearbeitet: Hela-Zellen (Beispiel 3), LTK-Mäusezellen (Fibro blasten; Beispiel 4) und humane FS4-Vorhautzellen (Fibroblast). Auch bei diesen Beispielen wurde die cytostatische und cytotoxische Wirkung des Ajoens bestätigt.

## Ansprüche

1. Verwendung von Verbindungen der allgemeinen Formel $R^1$-$CH_2$-SX-$CH_2$-$CR^2$ = $CR^3$-SY-SZ-$CH_2$-$R^4$

worin

$R^1$ und $R^4$ jeweils eine $C_{1-4}$-Alkyl-, Vinyl- oder Phenylgruppe sind,

$R^2$ und $R^3$ H-Atome sind oder mit den C-Atomen. an die sie gebunden sind, einen Phenylring bilden,

X ein oder zwei O-Atome bedeutet und

Y und Z unabhängig voneinander fehlen können oder ein O-Atom bedeuten,

als Cytostatika und/oder Cytotoxika oder in Cytostatika und/oder Cytotoxika.

2. Therapeutisches Mittel mit einem Gehalt an ein oder mehreren Verbindungen gemäß Anspruch 1 sowie einem physiologisch verträglichen Alkohol, beispielsweise Glyzerin, Ether und/oder Etheralkohol.

3. Therapeutisches Mittel nach Anspruch 2, **gekennzeichnet** durch einen Gehalt an üblichen Hilfsmitteln, insbesondere üblichen Trägern und/oder Verdünnungsmitteln, beispielsweise Wasser.

Hemmversuche an zwei Säugerzellinien (log Phase): Es ist die proz.
Hemmung des Thymidin-Einbaus in
Abhängigkeit von der Ajoen-Konzentration aufgezeichnet

o···o BHK (transformierte Baby-
Hamster-Kidney-Linie)
△--△ Humane Endothelialzellen

EP 0 353 416 A2